# FASCICULE DE BREVET EUROPEEN

(11) **EP 3 059 644 B1**
(45) Date de publication et mention de la délivrance du brevet: **16.08.2017**
(21) Numéro de dépôt: 16156214.5
(22) Date de dépôt: 17.02.2016
(51) Int. Cl.: G04G 9/00, G04G 21/02, A61B 5/16, A61B 5/00, G04F 10/00, A61B 5/053, A61B 5/11, A61B 5/145, A61B 5/0205, A61B 5/0476

(54) **DISPOSITIF PORTABLE ET PROCÉDÉ POUR LA DÉTERMINATION ET L'AFFICHAGE D'UNE DURÉE SUBJECTIVE**
TRAGBARE VORRICHTUNG UND VERFAHREN ZUR BESTIMMUNG UND ANZEIGE EINER SUBJEKTIVEN DAUER
PORTABLE DEVICE AND METHOD FOR DETERMINING AND DISPLAYING A SUBJECTIVE DURATION

(30) Priorité: 20.02.2015 CH 2312015
(43) Date de publication de la demande: 24.08.2016
(73) Titulaire: ECODT Sarl, 2000 Neuchatel (CH)
(72) Inventeur: Aubort Jaccard, Chantal, 2072 St-Blaise (CH)
(74) Mandataire: P&TS SA (AG, Ltd.)

(56) Documents cités:
- US-B1- 6 304 519

## Description

### Domaine technique

La présente invention concerne un dispositif et un procédé pour la détermination et l'affichage d'une durée subjective perçue par un utilisateur.

### Etat de la technique

L'horlogerie conventionnelle permet la mesure extrêmement précise du temps absolu, ou temps objectif, compté en secondes ou en multiples de la seconde. Il s'agit d'un temps collectif.

La présente invention concerne en revanche la mesure du temps subjectif, ou temps ressenti, c'est-à-dire l'évaluation du temps perçu par un individu et qui peut être différent du temps objectif. Il s'agit d'un temps individuel et personnel.

Il est en effet bien connu que la perception du déroulement du temps peut être variable ; parfois, le temps semble passer très vite alors qu'en d'autres moments il s'écoule beaucoup plus lentement.

Selon différentes études neurologiques, la perception de la vitesse d'écoulement du temps ne semble pas être associée à une zone sensorielle particulière du cerveau, mais dérive d'un système hautement distribué impliquant des portions du cortex cérébral, du cervelet et des ganglions de la base notamment, voire le coeur, les amygdales ou d'autres organes. Des systèmes différents sont responsables du rythme circadien (ou journalier) et de la mesure de durées plus brèves au cours de la journée. La coexistence de plusieurs horloges biologiques internes est probable et semble avérée par les études récentes.

La perception temporelle subjective est parfois étonnamment précise. Par exemple, un humain est capable de distinguer l'ordre de deux événements espacés de 20 millisecondes seulement. De nombreuses personnes sont capables de se réveiller chaque matin à une heure précise, par exemple exactement une minute avant que leur réveil ne sonne, même sans stimuli externes. La perception subjective de la durée de certains événements peut en revanche s'avérer peu précise ; par exemple, il est souvent difficile de se rappeler si un événement est survenu il y a cinq ans ou il y a dix ans. Sans horloge, il est aussi très difficile d'évaluer la durée d'une discussion, d'une partie de jeux ou d'une période de vacances par exemple.

La synchronisation entre le temps objectif et le temps subjectif, ou au contraire l'asymétrie perceptive, est la résultante de nombreux facteurs endogènes et exogènes. On sait ainsi que la perception subjective de la vitesse d'écoulement du temps est affectée par différents stimuli sensoriels externes, par exemple des stimuli visuels, auditifs, tactiles, etc. Le système sensoriel humain associe ces différents stimuli à d'autres événements pour les placer sur une échelle temporelle. Par exemple, la perception de l'évolution de la luminosité ambiante au cours de la journée a une influence directe sur le cycle circadien, comme les voyageurs sujets aux troubles de décalage horaire le savent bien.

On observe aussi que des paramètres physiologiques internes à la personne affectent sa perception du temps. La perception du temps semble s'accélérer lorsque la température corporelle monte, et se ralentir lorsque le corps se refroidit.

On sait par ailleurs qu'un état de stress peut introduire une hypersensibilité aux stimuli émotionnels et environnementaux, avec la capacité de discerner et de traiter plus d'informations par unité de temps, et donc fréquemment une perception du temps ralenti. Certaines personnes soumises à des états de stress extrême, par exemple en cas d'accident, disent ainsi « qu'une seconde a duré comme une année ». Différentes recherches ont aussi montré que le temps semble ralentir lorsque les personnes ressentent une menace ou une situation dangereuse, par exemple lors d'un plongeon vertigineux. La même sensation de décélération du temps survient lors d'événements imprévisibles.

Des émotions ou des états de relaxation, de détente, de joie, de confort etc modifient aussi la perception subjective du temps. Des recherches effectuées avec des étudiants en train de regarder des séquences de films d'angoisse ont révélé que ces étudiants ont souvent tendance à surestimer la durée des scènes. Certains biologistes pensent que cette illusion serait un mécanisme de défense impliquant les amygdales et permettant à la personne en état d'angoisse de ralentir son horloge interne afin de pouvoir prendre plus de décisions en moins de temps (augmenter la résolution temporelle).

La perception du temps peut être affectée par de nombreuses illusions ou filtres perceptifs, y compris l'effet Kappa. Cet effet conduit les personnes à surestimer une durée entre deux stimuli lorsque la distance spatiale entre ces stimuli est importante, et à sous-estimer cette même durée lorsque la distance spatiale est plus faible. Par exemple, un voyageur qui effectue deux trajets de même durée mais de longueur différente aura tendance à trouver que le voyage le plus lointain a duré plus longtemps que le court voyage de même durée.

L'âge de la personne a également une influence sur sa perception du temps; en vieillissant, on tend à sous-estimer davantage la durée des événements. Des différences d'évaluation de durée de près de 20% ont été observées entre un premier groupe de sujets entre 19 et 24 ans et un deuxième groupe entre 60 et 80 ans chargés d'évaluer une durée temporelle. Certains pensent aussi que l'activité mentale d'un enfant est en permanence plus élevée que celle d'un adulte, parce qu'un enfant découvre constamment des situations nouvelles pour lui et crée ainsi plus de souvenirs et plus d'émotions qu'un adulte soumis aux mêmes stimuli. Par conséquent, la perception subjective de la durée d'une période équivalente diminue avec l'âge. La durée subjective est aussi liée à « l'espace de stockage disponible » et à la capacité mémorielle de rétention.

Le corps ne semble pas seulement réagir à des stimuli exogènes ou endogènes en modifiant la perception du temps; il semble qu'il émette lui-même des signaux de changements physiologiques lorsque cette perception est modifiée et que le corps s'organise et adapte la temporalité. Par exemple, il semble qu'une sensation temporelle perturbée, comme on l'observe par exemple lors de décalages horaires, d'expérience de vies en grottes etc, crée en elle-même des signaux de stress, par exemple des hormones de stress, des altérations du rythme respiratoire et/ou cardiaque, des sudations, des modifications de la température corporelle, etc.

L'évaluation de durées temporelles subjectives, pouvant relever du réel, du symbolique et de l'imaginaire, est importante pour de nombreuses applications. Des marques de montre capables de telles mesures pourraient se distinguer de la concurrence en affichant une information supplémentaire, à la fois poétique et informative. Des objets industriels porteurs de sens pourraient ainsi être réalisés.

Des utilisateurs s'intéressent à obtenir une mesure chiffrée d'une perception ressentie, et à mesurer ainsi par exemple si leur journée de travail s'est écoulée plus ou moins rapidement. A l'inverse, des employeurs soucieux du bien-être de leurs employés pourraient mesurer leur perception du temps passé au travail, et en tenir compte pour offrir à chacun une position dans l'organisation qui lui offre une vitesse d'écoulement du temps optimale. La prise en considération rétrospective du temps ressenti offre à l'utilisateur la possibilité d'évaluer plus finement son temps prospectif pour planifier ses activités futures. Par exemple, un utilisateur pourrait déterminer qu'il a besoin d'un nombre relativement constant d'heures subjectives pour effectuer une tâche intellectuelle donnée, mais d'une durée objective variable. L'affichage de cette nouvelle mesure faciliterait le passage d'un temps imposé à un temps composé.

Des applications médicales, thérapeutiques et psychologiques bénéficieraient aussi d'une telle mesure du temps subjectif.

Différentes solutions ont donc été proposées dans l'art antérieur pour déterminer des durées temporelles subjectives. US6304519 décrit un dispositif pour comparer le temps réel qui s'est écoulé depuis un événement avec le temps subjectif perçu par l'utilisateur, et pour afficher la déviation. Le temps subjectif est déterminé sur la base de données introduites par l'utilisateur qui peut par exemple indiquer lui-même s'il effectue une activité ennuyeuse ou une activité pendant laquelle le temps s'écoule rapidement ou qui demande une implication intellectuelle plus importante. Le dispositif permet d'afficher la déviation entre le temps réel et le temps perçu, et d'afficher ces données en relation avec des paramètres biologiques tels que la température corporelle de l'utilisateur, son rythme cardiaque, la pression sanguine ou son métabolisme. Ce dispositif requiert donc impérativement la participation active de l'utilisateur qui doit indiquer lui-même le type d'activité dans laquelle il est engagé ou sa perception quant à l'écoulement du temps. Une telle indication manuelle est contraignante, et basée sur des évaluations subjectives qui ne permettent pas de comparer aisément les résultats de plusieurs utilisateurs.

US4630935 décrit un autre instrument permettant d'afficher des durées ressenties évaluées sur la base du nombre d'activations d'un actuateur manuel.

WO2007/107900A2 décrit une montre comportant un pointeur permettant d'afficher le degré de « coolness » d'un utilisateur pendant une durée passée. Ce degré de coolness est déterminé au moyen de paramètres physiologiques tels que le rythme cardiaque, la température corporelle, le mouvement, la résistance de la peau ou l'activité musculaire. Ce document n'explique pas la relation entre les paramètres physiologiques mesurés et le degré de « coolness » indiqué. Il n'y a pas non plus de relation entre le degré de « coolness » et la vitesse d'écoulement du temps.

Bien que l'on comprenne que la perception du temps ressenti soit affectée par des paramètres physiques mesurables, ou qu'elle entraîne des réactions physiologiques mesurables, la relation entre les différents paramètres mesurables et la durée ressentie est inconnue. Il n'existe donc pas de méthodes analytiques connues permettant de déterminer une durée subjective à partir d'un ensemble de grandeurs physiques ou chimiques.

### Bref résumé de l'invention

Un but de la présente invention est donc de proposer un dispositif portable capable de déterminer une durée subjective ressentie par un utilisateur de manière plus fiable que les dispositifs connus.

Selon l'invention, ces buts sont atteints notamment au moyen d'un dispositif portable sur le corps pour la détermination et l'affichage d'une durée subjective perçue par un utilisateur, comportant :
un ou plusieurs capteurs générant au moins un signal de mesure,
un indicateur permettant de représenter une durée ;
un processeur traitant ledit signal de mesure et commandant ledit indicateur,
une mémoire informatique stockant un module informatique exécutable par ledit processeur afin de commander ledit indicateur pour indiquer la durée subjective d'un intervalle temporel, en sorte que ladite durée subjective soit plus longue que la durée effective de cet intervalle lorsque ledit utilisateur a l'impression que le temps s'écoule lentement pendant ledit intervalle, et plus courte que la durée effective de cet intervalle lorsque ledit utilisateur a l'impression que le temps s'écoule rapidement pendant ledit intervalle,
ledit module informatique étant basé sur un système d'apprentissage automatique afin de déterminer ladite durée subjective sur la base dudit au moins un signal de mesure.

Dans un mode de réalisation, l'indicateur peut aussi indiquer un degré de satisfaction associé aux durées ressenties par l'utilisateur. L'indicateur peut par exemple indiquer si une accélération de la perception temporelle constitue une expérience satisfaisante pour l'utilisateur, ou au contraire un stress.

Par « dispositif portable sur le corps », on entend les dispositifs aussi souvent désignés par l'expression anglaise « wearable device ». Il s'agit donc de dispositifs destinés à être portés de manière continue ou quasi continue près du corps de l'utilisateur, sans l'empêcher de vaquer à ses occupations normales. Ces dispositifs sont portables sur le corps sans s'aider de ses mains. Des exemples de dispositifs portables sur le corps incluent par exemple des montres-bracelets, des lunettes intelligentes, des bracelets munis de capteur, des vêtements intelligents, des bijoux intelligents, etc.

La durée mesurée peut être entièrement dans le passé. Elle peut commencer dans le passé et se terminer à l'instant présent. Si la durée est brève, l'indicateur indique en chaque instant une vitesse d'écoulement du temps subjective à l'instant présent (ou pendant la brève période précédant l'instant présent).

Le système d'apprentissage automatique peut être basé par exemple sur un algorithme de machine learning. Il peut employer par exemple un classificateur auto-apprenant, par exemple un réseau neuronal, un réseau de machines à vecteurs de support (Support Vector Machines) et/ou un modèle de Markov caché.

L'utilisation d'un système d'apprentissage automatique permet de déterminer une durée subjective à partir de signaux de mesure variés, même sans connaître la relation analytique entre la valeur de ces signaux et la durée perçue.

Le système d'apprentissage automatique peut être supervisé, par exemple à l'aide d'une boucle de rétropertinence, ou non supervisé.

Le système d'apprentissage automatique peut être entraîné de manière propre à chaque utilisateur. Le temps subjectif affiché est ainsi propre et individuel à chaque utilisateur.

Le système d'apprentissage automatique peut être entraîné pour plusieurs utilisateurs à partir de données d'entraînement provenant d'un corpus d'utilisateurs, par exemple avec des indications de durées subjectives fournies par un corpus de plusieurs utilisateurs. Cela permet de disposer plus rapidement de davantage de données d'entraînement, et donc d'accélérer le processus d'entraînement.

Il est ainsi possible d'entraîner un système de manière universelle pour plusieurs utilisateurs. Il est possible d'améliorer cet entraînement universel initial en l'adaptant à la perception de chaque utilisateur individuel, ou de sous-groupes d'utilisateurs.

La durée subjective affichée peut être entièrement inscrite dans le passé. Le dispositif affiche alors la durée subjective d'un intervalle terminé, à la manière d'un chronographe qui affiche la durée d'une course terminée.

L'intervalle temporel dont on souhaite indiquer la durée subjective peut commencer dans le passé et se terminer à l'instant présent, en sorte que l'indicateur de durée subjective indique le ressenti instantané de la vitesse d'écoulement du temps.

Différents capteurs peuvent être employés afin de déterminer une durée subjective avec le dispositif de l'invention. Le rôle de chaque mesure dans la perception de la durée subjective n'a pas besoin d'être connu a priori ; le système d'apprentissage automatique détermine ce rôle de manière automatique. Toutefois, il est souhaitable de réduire le nombre de capteurs et de signaux de mesure, à la fois afin de réduire le coût du dispositif, son encombrement, sa consommation électrique, et pour rendre la classification des signaux de mesure plus rapide et plus robuste en renonçant aux signaux de mesure les moins pertinents.

Un ou plusieurs capteurs peuvent mesurer des paramètres exogènes à l'utilisateur, par exemple des paramètres environnementaux, comme la température ambiante, la luminosité, etc, ou des stimuli externes, par exemple des stimuli lumineux, des stimuli acoustiques, des stimuli tactiles, etc.

Un ou plusieurs capteurs peuvent mesurer des paramètres endogènes de l'utilisateur, par exemple sa température corporelle, son rythme cardiaque, sa pression artérielle, la résistivité de la peau, le taux de sudation, le taux de diverses hormones ou protéines sur la peau, et/ou l'élasticité de la peau.

Le système d'apprentissage automatique peut aussi utiliser des paramètres introduits par l'utilisateur, par exemple son âge, et/ou tirés de son agenda électronique, pour déterminer une durée subjective ressentie.

### Brève description des figures

Des exemples de mise en oeuvre de l'invention sont indiqués dans la description illustrée par les figures annexées dans lesquelles :
La figure 1 illustre une vue schématique d'un dispositif portable sur le corps selon l'invention.

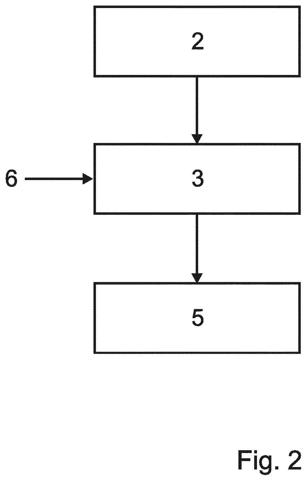

La figure 2 est un schéma-bloc d'un dispositif portable sur le corps selon l'invention.

### Exemple(s) de mode de réalisation de l'invention

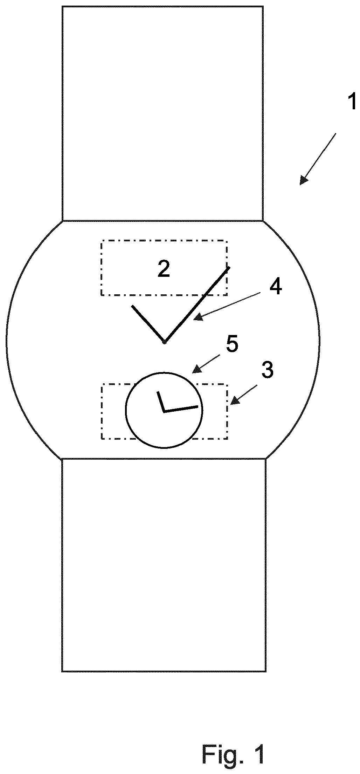

La figure 1 illustre de manière schématique un dispositif portable 1 selon l'invention, ici sous la forme d'une montre. Un dispositif similaire pourrait être intégré dans un bijou, un textile comme un maillot ou un gant par exemple, un bracelet, une ceinture, une combinaison entre plusieurs de ces dispositifs, etc.

Le dispositif comporte plusieurs capteurs 2 pour mesurer des paramètres exogènes, par exemple des grandeurs physiques environnementales, et/ou des grandeurs physiques endogènes de l'utilisateur. Un ou plusieurs des capteurs suivantes peuvent être prévus :
- Un capteur inertiel, par exemple un accéléromètre triaxial et/ou un gyroscope, afin de mesurer les déplacements de l'utilisateur ou d'une partie du corps de l'utilisateur. Une analyse de ces mouvements permet par exemple de déterminer l'activité à laquelle se livre l'utilisateur, ou de classer cette activité parmi des groupes d'activités. Il est par exemple possible de déterminer si l'utilisateur dort, marche, ou effectue un travail de bureau par exemple. L'utilisation d'un ou plusieurs capteurs inertiels permet aussi de déterminer la posture de l'utilisateur (assis, debout, couché), son activité ou des tremblements ou gestes nerveux caractéristiques par exemple. Le capteur inertiel peut être monté dans la montre, ou sur une autre partie du corps.
- Un récepteur de géolocalisation, par exemple un récepteur GPS, afin de déterminer l'emplacement de l'utilisateur, sa vitesse de déplacement, ou le moyen de déplacement employé. Ce récepteur de géolocalisation, et/ou le capteur inertiel, peut être utilisé pour déterminer la distance parcourue par l'utilisateur lors d'un déplacement ou entre deux stimuli, et pour évaluer ainsi l'influence de l'effet Kappa mentionné plus haut, en augmentant la durée du temps ressenti lors de déplacements de grande amplitude.
- Un capteur de température pour mesurer la température corporelle de l'utilisateur. La température corporelle peut en effet être un indicateur de l'activité physique et du stress de l'utilisateur.
- Un capteur de rythme cardiaque pour mesurer le pouls de l'utilisateur. Le rythme cardiaque peut en effet être un indicateur de l'activité physique et du stress de l'utilisateur.
- Un capteur permettant de mesurer un signal électrique à travers la peau de l'utilisateur, par exemple afin de mesurer la résistivité ou la capacitance de la peau, et donc son taux d'humidité par exemple. La sudation peut en effet être un indicateur de l'activité physique et du stress de l'utilisateur.
- Un capteur capable d'analyser la sueur de l'utilisateur, par exemple afin de mesurer la quantité de sueur ou sa composition, par exemple le taux de glycémie et/ou la présence d'hormones ou de protéines indicatrices du stress telle que la cortisone, l'adrénaline, la noradrénaline, la dopamine ou d'autres hormones ou protéines. Ce capteur peut être basé par exemple sur une plaquette microfluidique capable d'apporter par capillarité des échantillons de sueur sur des récepteurs.
- Un capteur mesurant le taux de testostérone sur la peau de l'utilisateur, comme indicateur d'activité.
- Un ou plusieurs capteurs environnementaux pour mesurer l'heure actuelle, la date actuelle, la température ambiante et/ou l'humidité ambiante et/ou la luminosité ambiante et/ou le niveau sonore ambiant. Il est aussi possible par exemple de distinguer entre différents types de signaux sonores afin de reconnaitre par exemple une discussion amicale ou une dispute, un bruit stressant, etc.
- Un module récupérant des données depuis l'agenda électronique et/ou depuis des réseaux sociaux de l'utilisateur, par exemple afin de déterminer à quelles activités se livre l'utilisateur à différents moments.
- Un capteur d'activité cérébrale, par exemple un détecteur d'électroencéphalogramme, capable par exemple de distinguer si le cerveau produit essentiellement des ondes α hautement périodiques, indiquant une activité cérébrale réduite, ou des ondes β aléatoires, indiquant une activité cérébrale plus intense.

Certains capteurs peuvent être présents dans la montre ou sur le boitier de la montre, par exemple dans son fond. Certains capteurs peuvent aussi être montés dans le bracelet, ou dans un autre dispositif porté ailleurs sur le corps, et connecté par exemple au travers d'une liaison sans fil avec la montre ou le dispositif incluant le processeur de traitement. Certains capteurs peuvent mesurer des données de manière continue, par exemple de manière continue pendant la mesure d'une durée relative, ou à intervalles réguliers, par exemple toutes les secondes, toutes les minutes etc, ou à la demande.

Le dispositif 1 peut aussi inclure des moyens d'entrée de données non représentés, par exemple un connecteur USB ou similaire, une interface radio de type Bluetooth ou similaire, un écran tactile, des boutons-poussoirs, etc, permettant à l'utilisateur d'introduire d'autres paramètres susceptibles d'influencer sa perception du temps. Dans un mode de réalisation, l'utilisateur peut ainsi indiquer son âge, son ethnicité ou s'il a consommé de l'alcool ou des substances psychogènes.

Le dispositif 1 inclut en outre des indicateurs 4 pour afficher l'heure courante (c'est-à-dire le temps absolu), ainsi qu'un ou des indicateurs 5 pour afficher le temps subjectif ressenti par l'utilisateur, comme on le verra plus loin. L'indicateur 5 peut être par exemple un indicateur analogique, par exemple un indicateur à aiguilles, ou un indicateur numérique, par exemple un indicateur alphanumérique ou un écran matriciel.

Un processeur 3 permet de recevoir le ou les signaux de mesure du ou des capteurs de mesure, et les données éventuelles introduites par l'utilisateur ou extraites de son agenda, afin de les traiter et de commander l'indicateur 5. Dans le cas d'un indicateur à aiguilles, le processeur 3 peut par exemple commander un moteur pas-à-pas entraînant les aiguilles. Dans le cas d'un entraîneur matriciel, le processeur peut par exemple générer des images fixes ou animées à afficher sur ledit écran.

Le processeur 3 exécute un programme comportant un module logiciel stocké dans une mémoire informatique afin de commander cet indicateur 5 pour indiquer la durée subjective d'un intervalle temporel, en sorte que ladite durée subjective soit plus longue que la durée effective de cet intervalle lorsque ledit utilisateur a l'impression que le temps s'écoule lentement pendant ledit intervalle, et plus courte que la durée effective de cet intervalle lorsque ledit utilisateur a l'impression que le temps s'écoule rapidement pendant ledit intervalle.

Le module informatique est basé sur un système d'apprentissage automatique afin de déterminer cette durée subjective sur la base des signaux de mesure des différents capteurs et/ou des paramètres introduits par l'utilisateur ou tirés de son agenda ou d'autres applications.

Dans un mode de réalisation, le système d'apprentissage automatique est basé sur un réseau neuronal entraîné pour classer des signaux de mesure reçus afin de déterminer une durée ressentie ou une vitesse subjective d'écoulement du temps.

Dans un autre mode de réalisation, le système d'apprentissage automatique est basé sur des réseaux de machines à vecteurs de support (Support Vector Machines, SVM) entraînés pour classer des signaux de mesure reçus afin de déterminer une durée ressentie ou une vitesse subjective d'écoulement du temps.

Dans un autre mode de réalisation, le système d'apprentissage automatique est basé sur des algorithmes de deep learning.

Dans un autre mode de réalisation, le système d'apprentissage automatique est basé sur un modèle de Markov caché (HMMs) ou des HMMs ergodiques afin d'analyser une séquence de comportements successifs.

L'apprentissage peut être effectué sur la base de mesures effectuées par lesdits capteurs et d'indications introduites par un ou plusieurs utilisateurs chargés de labéliser des durées, et d'indiquer si pendant ces durées le temps s'est écoulé rapidement ou lentement. Des notes ou des évaluations non binaires peuvent être introduites, par exemple pour indiquer une vitesse d'écoulement de -10 (très lent; le temps semble s'être arrêté) à +10 (très rapide). Le système d'auto-apprentissage reçoit les indications subjectives introduites par un utilisateur ainsi que les données de mesure fournies par les capteurs pendant les durées correspondantes, et s'entraîne ainsi à classifier des futures données de mesure.

Le système d'apprentissage automatique peut être paramétré avant son montage dans le dispositif, de manière indépendante de l'utilisateur, par exemple à partir de signaux de mesure et d'indications de données ressenties par un corpus d'utilisateurs. L'entraînement peut être effectué en demandant aux utilisateurs du corpus d'évaluer une durée subjective puis en introduisant cette évaluation ainsi que les paramètres mesurés correspondant dans le système d'apprentissage automatique.

Le système d'apprentissage automatique peut aussi être paramétré de manière individuelle à chaque utilisateur, par exemple au cours d'une séquence d'enrôlement explicite au cours de laquelle l'utilisateur indique la durée ressentie de différentes périodes au cours desquelles des mesures ont été prises au moyen des capteurs. Il peut aussi être paramétré en cours d'utilisation au moyen d'une boucle de rétropertinence, par exemple d'indications de durées ressenties par l'utilisateur, ou de corrections des estimations affichées par le dispositif 1. De cette manière, le système d'apprentissage automatique affiche à partir des mêmes signaux une durée subjective différente pour différents utilisateurs.

La durée subjective ressentie peut par exemple être affichée de manière analogique au moyen d'aiguilles 5, de manière similaire aux aiguilles d'un chronographe, afin d'afficher une durée ressentie depuis le déclenchement de la mesure au moyen d'un bouton poussoir par exemple, ou depuis un autre instant déterminé.

Il est aussi possible d'indiquer au moyen d'une seule aiguille ou d'un autre indicateur si la vitesse actuelle d'écoulement du temps est plus lente ou plus rapide que la vitesse objective.

Il est aussi possible d'afficher avec un indicateur le degré de satisfaction associé par l'utilisateur à une durée ou à un moment mesuré. Ce degré de satisfaction peut être déterminé à l'aide de mesure de paramètres endogènes.

Dans un mode de réalisation, la durée subjective est affichée sur un affichage matriciel, par exemple sous forme de donnée numérique, ou avec n'importe quelle autre représentation, par exemple sous forme de film animé. On pourrait par exemple afficher un cheval au galop lorsque le temps semble filer rapidement, et un animal plus lent lorsque le temps s'écoule paisiblement. Le choix du film peut dépendre de la vitesse d'écoulement au cours d'une période subjective située entièrement dans le passé, ou d'une période subjective continuant dans le présent; dans ce cas, le film peut représenter la vitesse d'écoulement du temps juste avant l'instant présent.

Des éléments du film ou de l'affichage matriciel, par exemple un paysage, un choix de personnage, un décor, un symbole etc, pourrait aussi représenter le degré de satisfaction ou de bien-être de l'utilisateur à chaque instant ou pendant une durée subjective mesurée.

Le décryptage sensoriel permet en outre de passer d'un temps descriptif à un temps prospectif voire indirectement prescriptif ; l'utilisateur est alors amené à co-construire le futur de sa mémoire et à mesurer son écosystème temporel par une opération d'ordre comparative. A l'usage d'une montre classique qui offre une fonction supplétive de la mémoire vient s'ajouter une lecture temporelle mentale individuelle. Un utilisateur peut alors par exemple prévoir le temps subjectif qui lui sera nécessaire pour accomplir ou terminer une tâche donnée. La montre peut aussi comporter un indicateur de compte à rebours du temps subjectif nécessaire pour l'accomplissement d'une tâche donnée.

La présente invention a aussi pour objet un support de données informatique incluant un programme informatique exécutable par un processeur pour la détermination et l'affichage d'une durée subjective perçue par un utilisateur, de manière à ce que le processeur exécute les opérations suivantes :
Réception d'au moins un signal de mesure généré au moyen d'un ou plusieurs capteurs;
Traitement du signal de mesure au moyen d'un système d'apprentissage automatique afin de déterminer une durée subjective sur la base dudit au moins un signal de mesure, en sorte que la durée subjective soit plus longue que la durée effective de cet intervalle lorsque l'utilisateur a l'impression que le temps s'écoule lentement pendant cet intervalle, et plus courte que la durée effective de cet intervalle lorsque l'utilisateur a l'impression que le temps s'écoule rapidement pendant cet intervalle,
Affichage de cette durée.

Ce programme informatique peut par exemple être destiné à être exécuté par un montre électronique, par exemple une montre connectée, ou par un téléphone portable intelligent.

## Revendications

1. Dispositif (1) portable sur le corps pour la détermination et l'affichage d'une durée subjective perçue par un utilisateur, comportant :
un ou plusieurs capteurs (2) générant au moins un signal de mesure,
un indicateur (5) permettant de représenter une durée;
un processeur (3) traitant ledit signal de mesure et commandant ledit indicateur,
une mémoire informatique stockant un module informatique exécutable par ledit processeur afin de commander ledit indicateur (5) pour indiquer la durée subjective d'un intervalle temporel, en sorte que ladite durée subjective soit plus longue que la durée effective de cet intervalle lorsque ledit utilisateur a l'impression que le temps s'écoule lentement pendant ledit intervalle, et plus courte que la durée effective de cet intervalle lorsque ledit utilisateur a l'impression que le temps s'écoule rapidement pendant ledit intervalle,
ledit module informatique étant **caractérisé en ce qu'**il est basé sur un système d'apprentissage automatique afin de déterminer ladite durée subjective sur la base dudit au moins un signal de mesure.

2. Dispositif selon la revendication 1, ledit système d'apprentissage automatique étant agencé pour déterminer à partir des mêmes signaux une durée subjective différente pour différents utilisateurs,
ledit système d'apprentissage automatique pouvant être entraîné de manière individuelle à l'utilisateur à l'aide d'une boucle de rétropertinence, par exemple de durées subjectives introduites ou corrigées par l'utilisateur.

3. Dispositif selon l'une des revendications 1 à 2, ledit système d'apprentissage automatique étant entraîné avec des indications de durées subjectives fournies par un corpus de plusieurs utilisateurs.

4. Dispositif selon l'une des revendications 1 à 3, ledit système d'apprentissage automatique étant basé sur un réseau neuronal, sur un modèle de Markov caché ou sur une machine à vecteurs de support.

5. Dispositif selon l'une des revendications 1 à 4, au moins un dit capteur étant un capteur inertiel, le processeur étant programmé pour déterminer ladite durée subjective en tenant compte des mouvements dudit utilisateur.

6. Dispositif selon l'une des revendications 1 à 5, au moins un dit capteur étant un capteur de température, le processeur étant programmé pour déterminer ladite durée subjective en tenant compte de la température corporelle dudit utilisateur.

7. Dispositif selon l'une des revendications 1 à 6, au moins un dit capteur étant agencé pour mesurer une grandeur électrique liée à un signal électrique à travers la peau dudit utilisateur, le processeur étant programmé pour déterminer ladite durée subjective en tenant compte de ladite grandeur électrique.

8. Dispositif selon l'une des revendications 1 à 7, au moins un dit capteur étant agencé pour mesurer la sueur sur la peau dudit utilisateur, le processeur étant programmé pour déterminer ladite durée subjective en tenant compte de ladite sueur.

9. Dispositif selon l'une des revendications 1 à 8, au moins un dit capteur étant agencé pour mesurer le taux d'une hormone ou protéine sur la peau dudit utilisateur, le processeur étant programmé pour déterminer ladite durée subjective en tenant compte dudit taux d'hormone ou de protéine.

10. Dispositif selon l'une des revendications 1 à 9, au moins un dit capteur étant agencé pour mesurer la température ambiante et/ou l'humidité ambiante et/ou la luminosité ambiante, le processeur étant programmé pour déterminer ladite durée subjective en tenant compte de la température ambiante et/ou l'humidité ambiante et/ou la luminosité ambiante.

11. Dispositif selon l'une des revendications 1 à 10, ledit processeur étant programmé pour déterminer ladite durée subjective en tenant compte d'événements inscrits dans un agenda de l'utilisateur.

12. Dispositif selon l'une des revendications 1 à 11, ledit module informatique exécutable par ledit processeur permettant de commander un indicateur pour indiquer un degré de satisfaction dudit utilisateur pendant ledit intervalle temporel.

13. Dispositif selon l'une des revendications 1 à 12, constitué par une montre-bracelet.

14. Procédé pour la détermination et l'affichage d'une durée subjective perçue par un utilisateur, comportant :
Génération d'au moins un signal de mesure au moyen d'un ou plusieurs capteurs dans un dispositif portable ;
Traitement dudit signal de mesure au moyen d'un processeur, ledit processeur exécutant un module informatique basé sur un système d'apprentissage automatique afin de déterminer une durée subjective sur la base dudit au moins un signal de mesure, en sorte que ladite durée subjective soit plus longue que la durée effective de cet intervalle lorsque ledit utilisateur a l'impression que le temps s'écoule lentement pendant ledit intervalle, et plus courte que la durée effective de cet intervalle lorsque ledit utilisateur a l'impression que le temps s'écoule rapidement pendant ledit intervalle,
affichage de ladite durée.

15. Support de données informatique incluant un programme informatique exécutable par un processeur pour la détermination et l'affichage d'une durée subjective perçue par un utilisateur, de manière à ce que le processeur exécute les opérations suivantes :
Réception d'au moins un signal de mesure généré au moyen d'un ou plusieurs capteurs;
Traitement dudit signal de mesure au moyen système d'apprentissage automatique afin de déterminer une durée subjective sur la base dudit au moins un signal de mesure, en sorte que ladite durée subjective soit plus longue que la durée effective de cet intervalle lorsque ledit utilisateur a l'impression que le temps s'écoule lentement pendant ledit intervalle, et plus courte que la durée effective de cet intervalle lorsque ledit utilisateur a l'impression que le temps s'écoule rapidement pendant ledit intervalle,
Affichage de ladite durée.

## Patentansprüche

1. Vorrichtung (1), die an einem Körper tragbar ist, zum Bestimmen und Darstellen einer subjektiv von einem Benutzer empfundenen Dauer, aufweisend:
einen oder mehrere Sensoren (2), der oder die mindestens ein Messsignal erzeugt oder erzeugen,
eine Anzeige (5), der die Darstellung einer Dauer erlaubt;
einen Prozessor (3), der das Messsignal verarbeitet und die Anzeige steuert,
einen Computerspeicher, der ein Computermodul speichert, das von dem Prozessor ausführbar ist, um die Anzeige (5) anzusteuern, die subjektive Dauer eines Zeitintervalls so anzuzeigen, dass die subjektive Dauer eines Zeitintervalls länger als die tatsächliche Dauer dieses Zeitintervalls ist, wenn der Benutzer den Eindruck hat, dass die Zeit während dieses Intervalls langsam vergeht, und kürzer als die tatsächliche Dauer dieses Zeitintervalls ist, wenn der Benutzer den Eindruck hat, dass die Zeit während dieses Intervalls schnell vergeht,
wobei das Computermodul **dadurch gekennzeichnet ist, dass** es auf einem automatischen Lernsystem basiert, um die subjektive Dauer auf der Basis von dem mindestens einem Messsignal zu bestimmen.

2. Vorrichtung nach Anspruch 1, wobei das automatische Lernsystem ausgebildet ist, um von dem gleichen Signal eine unterschiedliche subjektive Dauer für unterschiedliche Benutzer zu bestimmen,
wobei das automatische Lernsystem ausgebildet ist, in individueller Weise für jeden Benutzer durch eine Feedbackschleife trainiert zu werden, zum Beispiel mit subjektiven Dauern, die von dem Benutzer eingegeben oder korrigiert werden.

3. Vorrichtung nach einem der Ansprüche 1 bis 2, wobei das automatische Lernsystem durch Anzeigen einer subjektiven Dauer, die von einer Menge von verschiedenen Benutzern geliefert werden, trainiert wird.

4. Vorrichtung nach einem der Ansprüche 1 bis 3, wobei das automatische Lernsystem auf neuronalen Netzwerken, auf Hidden Markov Modellen oder auf einer Support Vector Machine basiert.

5. Vorrichtung nach einem der Ansprüche 1 bis 4, wobei der mindestens eine Sensor ein Inertialsensor ist, wobei der Prozessor programmiert ist die subjektive Dauer unter Berücksichtigung der Bewegungen des Benutzers bestimmt.

6. Vorrichtung nach einem der Ansprüche 1 bis 5, wobei der mindestens eine Sensor ein Temperatursensor ist, wobei der Prozessor programmiert ist, die subjektive Dauer unter Berücksichtigung der Körperwärme des Benutzers zu bestimmen.

7. Vorrichtung nach einem der Ansprüche 1 bis 6, wobei der mindestens eine Sensor ausgebildet ist, eine elektrische Grösse bezüglich einem elektrischen Signal durch die Haut des Benutzers zu messen, wobei der Prozessor programmiert ist, die subjektive Dauer unter Berücksichtigung der elektrischen Grösse zu bestimmen.

8. Vorrichtung nach einem der Ansprüche 1 bis 7, wobei der mindestens eine Sensor ausgebildet ist, den Schweiss auf der Haut des Benutzers zu messen, wobei der Prozessor programmiert ist, die subjektive Dauer unter Berücksichtigung des Schweiss zu bestimmen.

9. Vorrichtung nach einem der Ansprüche 1 bis 8, wobei der mindestens eine Sensor ausgebildet ist, das Niveau eines Hormons oder Proteins auf der Haut des Benutzers zu messen, wobei der Prozessor programmiert ist, die subjektive Dauer unter Berücksichtigung des Niveaus des Hormons oder Proteins zu bestimmen.

10. Vorrichtung nach einem der Ansprüche 1 bis 9, wobei der mindestens eine Sensor ausgebildet ist, die Umgebungstemperatur and/oder - feuchtigkeit und/oder - licht zu messen, wobei der Prozessor programmiert ist, die subjektive Dauer unter Berücksichtigung der Umgebungstemperatur and/oder -feuchtigkeit und/oder - licht zu bestimmen.

11. Vorrichtung nach einem der Ansprüche 1 bis 10 wobei der Prozessor so programmiert ist, um die subjektive Dauer unter Berücksichtigung von Events zu bestimmen, die in dem Kalender des Benutzers registriert sind.

12. Vorrichtung nach einem der Ansprüche 1 bis 11, wobei das Computermodul, das von dem Prozessor ausführbar ist, die Steuerung einer Anzeige erlaubt, um das Niveau der Genugtuung des Benutzers während des Zeitintervalls anzuzeigen.

13. Vorrichtung nach einem der Ansprüche 1 bis 12, bestehend aus einer Armbanduhr.

14. Verfahren zum Bestimmen und Darstellen einer subjektiv von einem Benutzer empfundenen Dauer, aufweisend:
Erzeugen mindestens ein Messsignals mittels eines oder mehrerer Sensoren in einem tragbaren Gerät,
Verarbeiten des Messsignals in einen Prozessor, wobei der Prozessor ein Computermodul ausführt, das auf einem automatischen Lernsystem basiert, um die subjektive Dauer auf der Basis von dem mindestens einem Messsignal zu bestimmen, so dass die subjektive Dauer länger als die tatsächliche Dauer dieses Zeitintervalls ist, wenn der Benutzer den Eindruck hat, dass die Zeit während dieses Intervalls langsam vergeht, und kürzer als die tatsächliche Dauer dieses Zeitintervalls ist, wenn der Benutzer den Eindruck hat, dass die Zeit während dieses Intervalls schnell vergeht,
Darstellen der Dauer.

15. Computerdatenträger mit einem Computerprogramm ausführbar von einem Prozessor zum Bestimmen und Darstellen einer von eimem Benutzer empfundenen subjektiven Dauer, so dass der Prozessor die folgenden Operationen ausführt:
Empfangen mindestens ein Messsignals, das/die mittels eines oder mehrerer Sensoren erzeugt wird/werden,
Verarbeiten des Messsignals in einen Prozessor, wobei der Prozessor ein Computermodul ausführt, das auf einem automatischen Lernsystem basiert, um die subjektive Dauer auf der Basis von dem mindestens einem Messsignal zu bestimmen, so dass die subjektive Dauer länger als die tatsächliche Dauer dieses Zeitintervalls ist, wenn der Benutzer den Eindruck hat, dass die Zeit während dieses Intervalls langsam vergeht, und kürzer als die tatsächliche Dauer dieses Zeitintervalls ist, wenn der Benutzer den Eindruck hat, dass die Zeit während dieses Intervalls schnell vergeht,
Darstellen der Dauer.

## Claims

1. Device (1) wearable on the body for determining and displaying a subjective duration perceived by a user, comprising :
one or several sensors (2) generating at least one measuring signal,
an indicator (5) allowing the representation of a duration;
a processor (3) processing said measuring signal and commanding said indicator,
a computer memory storing a computer module executable by said processor in order to command said indicator (5) to indicate the subjective duration of a time interval, in such a manner that said subjective duration is longer than the actual duration of this interval when said user has the impression that the time passes slowly during said interval, and shorter than the actual duration of said interval when said user has the impression that time is passing quickly during said interval,
said computer module being **characterized in that** it is based on an automatic learning system in order to determine said subjective duration on the basis of said at least one measuring signal.

2. Device according to claim1, said automatic learning system being set up in order to determine from the same signals a different subjective duration for different users,
said automatic learning system being able to be trained in an individual manner for the user by means of a feedback loop, for example with subjective durations introduced or corrected by the user.

3. Device according to one of the claims 1 to 2, said automatic learning system being trained by indications of subjective duration supplied by a set of several users.

4. Device according to one of the claims 1 to 3, said automatic learning system being based on a neural network, on a hidden Markov model or on a support vector machine.

5. Device according to one of the claims 1 to 4, at least one said sensor being an inertial sensor, the processor being programmed to determine said subjective duration taking into account movements of said user.

6. Device according to one of the claims 1 to 5, at least one said sensor being a temperature sensor, the processor being programmed to determine said subjective duration taking into account the body temperature of said user.

7. Device according to one of claims 1 to 6, at least one said sensor being arranged to measure an electrical quantity related to an electric signal through the skin of said user, the processor being programmed to determine said subjective duration while taking into account said electrical quantity.

8. Device according to one of claims 1 to 7, at least one said sensor being arranged to measure the sweat on the skin of said user, the processor being programmed to determine said subjective duration taking into account said sweat.

9. Device according to one of claims 1 to 8, at least one said sensor being arranged to measure the level of a hormone or protein on the skin of said user, the processor being programmed to determine said subjective duration taking into account said level of hormone or protein.

10. Device according to claims 1 to 9, at least one said sensor being set up to measure the ambient temperature and/or the ambient humidity and/or the ambient light, the processor being programmed to determine said duration taking into account the ambient temperature and/or the ambient humidity and/or the ambient light.

11. Device according to one of claims 1 to 10, said processor being programmed to determine said subjective duration while taking into account events registered in the calendar of the user.

12. Device according to one of the claims 1 to 11, said computer module executable by said processor allowing the command of an indicator to indicate the level of satisfaction of said user during said time interval.

13. Device according to one of the claims 1 to 12, consisting of a wrist-watch.

14. Process for determining and displaying a subjective duration perceived by a user, including:
Generation of at least one measuring signal by means of one or several sensors in a portable device;
Processing of said measuring signal by means of a processor, said processor executing a computer module based on an automatic learning system to determine the subjective duration on the basis of said at least one measuring signal in such a way that said subjective duration is longer than the effective duration of this interval when said user has the impression that time is passing slowly during said interval, and shorter than the effective duration of this interval when said user has the impression that time is passing quickly during said interval,
display of said duration.

15. Computer data carrier including a computer programme executable by a processor for determining and displaying a subjective duration perceived by a user, so that the processor executes the following operations:
Receiving at least one measuring signal generated by means of one or several sensors;
Processing of said measuring signal by means of an automatic learning system in order to determine a subjective duration based on said at least one measuring signal, so that said subjective duration is longer than the effective duration of this interval when said user has the impression that time is passing slowly during said interval, and shorter than the effective duration of this interval when said user has the impression that time is passing quickly during said interval,
Display of said duration.
